# EUROPEAN PATENT APPLICATION

(11) **EP 2 586 481 A1**
(43) Date of publication of application: **01.05.2013**
(21) Application number: 11186984.8
(22) Date of filing: 27.10.2011
(51) Int. Cl.: A61M 16/20, F17C 13/06

(54) **Cover for a valve cap of a gas cylinder**

(71) Applicant: Linde AG, 80331 München (DE)
(72) Inventor: Cesbron, Anthony, 78520 Limay (FR); Planes, Abdon, 60200 Compiegne (FR)
(74) Representative: Dörries, Hans Ulrich

(57) **Abstract**

The present invention pertains to a cover (3) for a valve cap (2) of a gas cylinder (1), wherein the cover (3) includes a cavity (340, 342) which is dimensioned to receive portions of the valve cap (2) of the gas cylinder (1), wherein the cover (3) is made from a substantially rigid material.

## Description

### Technical Field

The present invention pertains to a cover for a valve cap of a gas cylinder, preferably to a cover for a valve cap of a portable gas cylinder containing medical gases.

### Technological Background

In the field of the distribution of medical gases it is known to distribute gases by means of gas cylinders. Specialty medical gases such as helium or nitrous oxide or standard medical gases such as oxygen and medical air are distributed to the respective hospitals or treatment centers in gas cylinders. In an alternative distribution channel, the gas cylinders are directly delivered to the home of a patient in order to be used in homecare applications. The gas cylinders are then either connected to a piping system in the hospital or in an emergency vehicle, or are directly used at the administration site, for example directly at a patient's bed.

In the latter case, the gas cylinders are preferably relatively small such that they are easily portable. The provision of portable gas cylinders obviates the need to provide gas outlets of a hospital pipeline system at the patient's bed and/or offers the possibility to relocate the respective patient easily within the hospital without interrupting the gas supply because the gas cylinder can be moved together with the bed of the patient. Furthermore, portable gas cylinders can be easily suspended in the vicinity of the bed of the patient by means of a hook provided at the gas cylinder which can be connected to a structure of the patient's bed.

The gas cylinders which are used in the medical field are typically fitted with a specific valve structure which is fixedly secured to the outlet portion of the gas cylinder such that the user can start administering the gas from the gas cylinder immediately without the need for mounting any valve components to the gas cylinder such as a pressure regulator. At the same time, the supplier of the gas cylinder can be sure that the valve structure is tailored to the specific specifications of the respective gas. In order to improve the mechanical integrity and in order to safeguard the delicate valve structure, the gas cylinder usually carries a valve cap around the valve structure wherein this valve cap is typically fixedly attached to the gas cylinder and/or to the valve structure.

Such a conventional valve cap of a gas cylinder usually has several openings through which the valve structure can be accessed such that a gas outlet and a hand wheel for opening and closing the valve are accessible whilst the valve cap remains fixedly attached to the gas cylinder. In preferred embodiments of these conventional caps, the openings are formed such that a user is additionally enabled to read a manometer and/or to connect a gas conduit to a separate low pressure gas outlet.

It should be noted that if a valve structure of a gas cylinder should ever be broken off whilst the gas is still present under high pressure in the cylinder, the gas in the cylinder would, upon escaping, tend to give the gas cylinder rocket propulsion. As this condition is very dangerous and needs to be avoided by any means, the above-mentioned valve caps of the gas cylinders are fixedly attached to the gas cylinders or valve structures and are dimensioned to provide adequate mechanical resistance against the occurrence of damages to the valve structure.

In addition, conventional valve caps may serve as a handle for conveniently carrying the gas cylinder. In order to serve as a handle, the valve cap may comprise a specific handle portion such as - for example - a cutout in the wall of the valve cap which enables a user to easily carry the portable gas cylinder. In a further embodiment of the conventional valve caps, it is additionally supplied with a hook which is intended to suspend the gas cylinder from a respective structure.

Such a standard valve cap for a portable gas cylinder, in particular a gas cylinder containing oxygen, is described, for example, in US 5,429,152. The valve cap is intended to remain on the gas cylinder and cannot be removed from the gas cylinder.

However, valve caps which are conventionally used for safeguarding and protecting the valve structure necessarily have certain openings which are used to access the respective gas outlets, hand wheels for operating the valve structure as well as observing manometers of the valve structure. Accordingly, the valve structure is always open such that dust and dirt may easily enter and compromise the functionality of the gas cylinder and/or may contaminate the gas product stored in the gas cylinder itself.

In order to avoid contamination of the valve structure it has been common practice to cover the valve structure of the gas cylinder by placing a plastic bag, a shrink bag or a shrink film around it in order to avoid dust and moisture to enter the valve structure. However, placing the films around the valve structure is cumbersome and cannot be easily automated. Furthermore, it is difficult for the end user to remove the films before using such that this solution does not prove comfortable for the end user.

In addition, due to the open structure of the valve cap, it cannot be controlled whether the gas cylinder has been tampered with before it is actually used for the first time to administer the gas to a patient. Accordingly, it is attempted to seal-off the valve structure by means of the aforementioned plastic bag, shrink bag or shrink film.

Such a tamper-proof arrangement is also of relevance for all other gases such as industrial gases in order to make sure that the customer receives the gas in the form as originally filled into the gas cylinder by the supplier.

### Summary

Accordingly, it is an objective of the present invention to improve the reliability of the valve structure in a gas cylinder having a valve cap.

The above-mentioned objective is solved by means of a cover for a valve cap of a gas cylinder with the features of claim 1. Advantageous embodiments of the invention are mentioned in the dependent claims.

Accordingly, the cover for a valve cap of a gas cylinder includes a cavity which is dimensioned to receive at least portions of the valve cap of the gas cylinder. According to the invention, the cover is made from a substantially rigid material.

In the cavity, portions of the valve cap can be received. However, depending on the geometry of the valve cap it is also contemplated that the entire valve cap is received in the cavity.

By means of the provision of a cover which is made from a substantially rigid material which covers at least portions of the valve cap of the gas cylinder, it becomes possible to improve the reliability of the valve structure because the valve cap of the gas cylinder is prevented from becoming contaminated and tampered with because it is surrounded by the cover.

The provision of the rigid structure greatly affects the applicability of the cover to the valve cap such that this can be done in an automated manner - or at least with a significant reduction of manual labor. The cover can be placed around the valve cap and covers not only the valve cap but - by securely covering the openings in the valve cap - also covers the valve structure. Because the material of the valve cap is substantially rigid, the cover provides a robust structure even when extending over the openings which are provided in the valve cap. Furthermore, because it is made from a rigid material, it will not be possible for a third party to tamper with the gas cylinder without this attempt being noticed. The rigid structure cannot easily be moved away in order to access the hand wheel and/or the gas outlets unlike in the case of a flexible plastics bag, shrink bag or shrink film. Furthermore, the rigid cover prohibits any third party to access the openings in the valve cap and, thus, to reach with their hands and/or tools within the valve cap to tamper with the valve structure.

In this context it is to be noted that the valve cap which covers the valve unit of the gas cylinder is often also referred to as a "guard", a "cap" or a "Valve Protection Carry Handle" (VPCH).

The cover is preferably made from a thermoformable material, preferably a thermoformable plastics material, and is preferably made from PET (Polyethylene Terephthalate), PVC (Polyvinyl Chloride), PCTFE (Polychlorotrifluoro ethylene) laminated to PVC, COC (Cyclic Olefin Copolymers), COP (Cyclic Olefin Polymers), preferably in multilayered combinations with PP (Polypropylene), PE (Polyethylene), or PETg (glycol-modified polyethylene terephthalate). By the provision of this material, a cost effective production of the cover can be achieved. With this type of material it is also possible to manufacture the cover such that it closely resembles the outer proportions of the valve cap of the gas cylinder. The manufacturing process of molding and trimming a thermoformable plastics sheet such as to make a final product is well-known such that the cover can be manufactured easily, reliably and economically. In fact, the thermoforming process is even more economical than an injection process. Nevertheless, the cover could also be manufactured using an injection molding process such that the cover is injection molded.

Preferably, the cover is provided in the form of a blister pack and/or a clamshell pack. These forms of packages enable an efficient production of the cover while ensuring that the integrity of the valve structure within the valve cap is maintained as long as the valve cap is received in the cover.

In a preferred embodiment of the present invention, the cover comprises two half-shells which form the cavity for receiving portions of the valve cap when folded upon themselves At the same time, the provision of two half-shells enables the cover to be formed to closely resemble the outer contours of the valve cap already because undercuts can be formed more easily.

In order to further improve the tamper-proof characteristics of the cover, the two half-shells are preferably interconnectable by means of at least one tamper-proof connector to form a tamper-proof cover. The tamper-proof connector is preferably made such that it can only be connected once and such that it is destroyed as soon as it is attempted to open the once closed connection again. By means of this tamper-proof connector it is ensured that a user can easily determine whether the gas cylinder is in the intended state, i.e. in the state it has left the facilities of the respective supplier, or whether the gas cylinder has been tampered with. This is of importance for all different fillings of a gas cylinder such as for medical gases, industrial gases as well as any other gases.

Preferably, the two half-shells are connected via a foil hinge. The provision of a foil hinge enables even easier application of the cover to the valve cap because it can be easily folded around the valve cap.

Preferably, the two half-shells can be interconnected by means of a tamper-proof connector, such as a clip, a thermal welding, an agrafe, a cramp, a staple, a plastic collar and/or an adhesive band, such that the cover can effectively be applied around the valve cap of the gas cylinder but cannot be removed anymore without being destroyed. When such a cover is applied to the valve cap once the gas cylinder has been filled at a filling site of a gas supplier or gas distributor, the end-user, e.g. the employee in the hospital, the end-user in a homecare application or the end user in an industrial application, can easily determine whether the gas cylinder is in its originally filled state or whether it has been tampered with. When the cover is fully intact and in place, the end-user can be certain that the gas cylinder and its contents have not been tampered with. Furthermore, the end-user can also be certain that the filling amount of the gas cylinder has not changed because it was not possible to release gas from the gas cylinder without having access to the valve structure.

In an alternative, the cover comprises more than two parts which form the cavity when connected. By means of providing not only two half-shells but more than two parts of the cover for forming the cavity to receive at least portions of the valve cap, the cover may be adapted to specific geometries of the valve cap more easily. In particular, the division line between the respective parts of the cover can be defined such that the cover can be easily applied around the valve cap.

Preferably, the at least two parts are interconnectible by means of at least one tamper-proof connector to form a tamper-proof cover and/or wherein at least two parts are connected via a foil hinge and/or a tongue.

In a further preferred embodiment, the cavity is formed such that it fully surrounds the valve cap of the gas cylinder when applied to the valve cap. By fully enclosing the valve cap of the gas cylinder, the safety standard is further raised. Furthermore, by fully covering the valve cap of the gas cylinder, contamination of the valve structure situated inside the valve cap of the gas cylinder can be avoided.

The cover may be made in one piece, preferably by thermoforming a thermoformable plastics sheet, in order to increase its reliability and in order to reduce the cost of manufacturing. Of course, the one-piece cover could also be made by injection molding.

To be in a position to easily carry the gas cylinder, it is preferred that a handle portion is formed in the cavity, wherein this handle portion of the cover resembles a handle portion of the valve cap. Accordingly, a user can carry the gas cylinder by carrying it at the handle portion which is provided at the valve cap by gripping the handle portion of the cover. However, at the same time the contents of the valve cap are still secured because the cover is present as an outer layer. In other words, the user can determine whether the cover is still intact or whether the contents of the gas cylinder have been tampered with.

In order to make surer that the cover is securely fitted to the gas cylinder, preferably a collar is formed in the cover which is intended to be arranged at a bottom portion of the valve cap. In an even more preferred embodiment, the collar defines an opening which has a diameter which is smaller than the largest diameter of the valve cap. By the provision of this collar it will become impossible to remove the cover from the valve cap by simply pulling it away from the gas cylinder without breaking at least the collar off the cover. Preferably, the collar is intended to encircle the valve unit between the valve cap and the actual gas cylinder such that the cover, in its closed and applied state, cannot be removed from the valve cap without being destroyed.

In yet another preferred embodiment, the cover is formed to hermetically enclose the valve cap of the gas cylinder when it is being applied to the valve cap. By means of this hermetic enclosure it is made sure that no third party can access any relevant portions of the valve structure without removing and - thus - destroying the cover.

The cavity defined by the cover is preferably suitable for receiving the valve cap of the gas cylinder in its entirety and the contours of the inside of the inner cavity preferably resemble the outer contours of the valve cap of the gas cylinder such that the cover sits closely and firmly on the valve cap of the gas cylinder. This has the advantage that contamination of the valve cap of the gas cylinder as well as the valve unit situated inside the valve cap can be safely avoided, and that it is avoided that the rigid cover is destroyed once the gas cylinder is tossed around because the wall thickness of the cover is not of relevance anymore but it is rather completely supported by the wall of the valve cap as such.

Preferably, the cover covers all openings of the valve cap of the gas cylinder such that contamination and inadvertent operation and tampering with the valve unit located inside the valve cap but usually accessible through the openings in the valve cap can be surely avoided.

In another preferable embodiment, the cover comprises a cavity which closely resembles or fits into the contours of a handle in the valve cap of the gas cylinder. By means of the provision of such a cavity it becomes possible carrying the gas cylinder in one hand even if the cover is still in place.

Preferably, the cover includes openings and/or cutouts for receiving a hook or any other fastening means situated at the valve cap of the gas cylinder.

### Brief Description of the Figures

In the following, the present disclosure is further exemplified by means of the Figures of the drawings in which:
- Figure 1: is a schematic, perspective view of an exemplary cover placed on a valve cap of a gas cylinder;
- Figure 2: is a schematic, perspective view of the cover for a valve cap of a gas cylinder in an opened state, showing the inner cavity of the cover;
- Figure 3: is a schematic, perspective view of the cover in a closed condition;

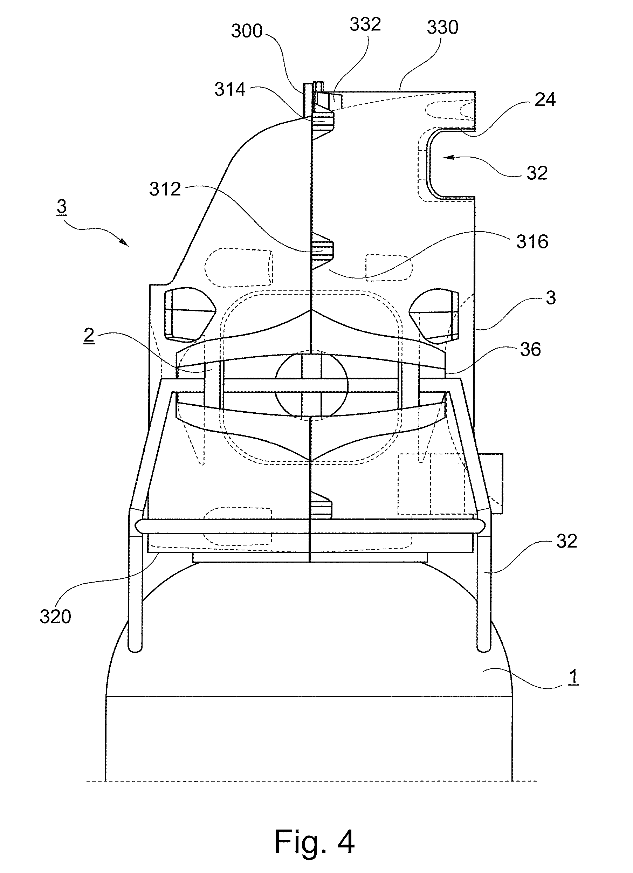

- Figure 4: is a schematic side view of the cover placed on a valve cap of a gas cylinder; and
- Figure 5: is a schematic perspective view of the opened cover of Figure 2 taken from a different perspective.

### Detailed Description of the preferred Embodiments

In the following discussion of the preferred embodiments, similar elements are identified with identical reference numerals, and repeated description of the respective elements may be omitted in order to avoid redundancies in the specification.

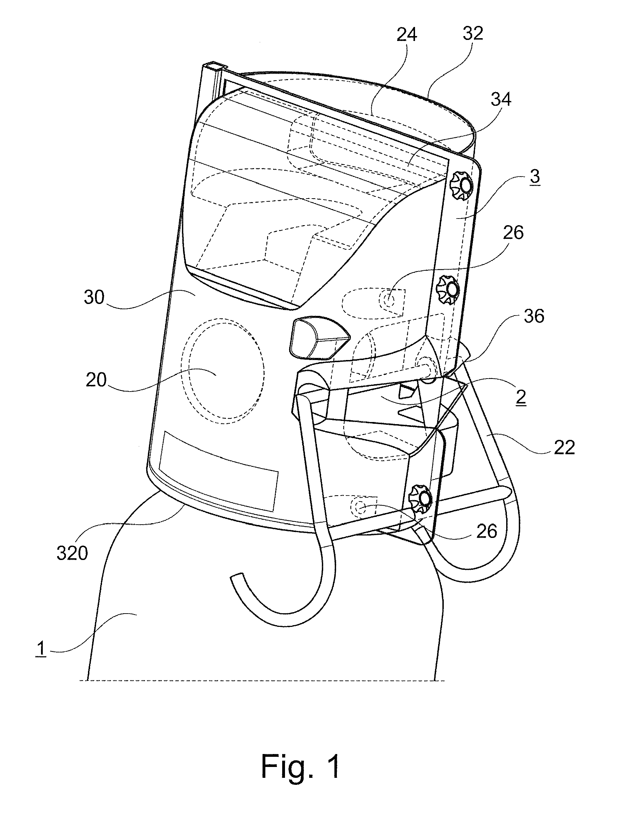

Figure 1 shows, in a schematic perspective view, an assembly of a gas cylinder 1 with a valve cap 2 wherein the valve cap 2 is intended to enclose a valve structure or valve unit associated with the gas cylinder 1. In Figure 1, this valve structure is not explicitly shown but it is assumed to be located inside of the valve cap 2. The valve cap 2 is fixedly attached to the gas cylinder 1, e.g. by means of a set of screws 26 of the valve cap 2. In the embodiment shown in Figure 1, the valve cap 2 comprises at least two half-shells which are connected by means of the screws 26 such that a clamping action and/or form fit between valve cap 2 and gas cylinder 1 is achieved such that the valve cap 2 is fixedly attached to the gas cylinder 1.

A cover 3 for covering the valve cap 2 of the gas cylinder 1 is provided, wherein the cover 3 includes a rigid material and forms a cavity for receiving the valve cap 2 of the gas cylinder 1. In the embodiments shown in the Figures, cover 3 comprises at least two half-shells 30, 32, wherein the two half-shells 30, 32 form the cavity when they are connected to one another such that the valve cap 2 can be fully received in the cavity.

The gas cylinder 1 is preferably a portable gas cylinder which is typically used in homecare applications and emergency applications in hospitals or to supply gas when a patient is transported within a hospital. In order to qualify to be portable, the volume of the gas cylinder 1 is preferably in a range of between 1 and 15 liters such that the weight of the gas cylinder 1 can still be handled by medical personnel, preferably singlehanded.

The valve cap 2 is fixedly attached to the gas cylinder 1 and is intended to safeguard and protect a valve unit of the gas cylinder 1. The valve unit is fully received inside of the volume defined by the valve cap 2 and serves to output gas from the gas cylinder 1 to the respective users. The valve unit, therefore, includes at least one gas outlet and typically at least one hand-wheel for opening and closing the valve. In preferred embodiments, the valve unit furthermore includes a low pressure output wherein the low pressure is achieved by means of an internal pressure regulator. In further preferred embodiments, a manometer is provided in order to monitor the pressure of the gas received in the gas cylinder 1.

In order to access the respective gas outputs, hand-wheels and manometers, openings 20 are present in the valve cap 2. Through these openings 20 the different elements of the valve unit can be accessed by a user in order to operate the gas cylinder 1.

A hook 22 is also present and is directly attached to the valve cap 2, wherein the hook 22 is intended to hang the gas cylinder 1 to a structure while in use.

The valve cap 2, furthermore, comprises a handle portion 24 which allows a user to carry the gas cylinder 1 conveniently by simply gripping the handle portion 24 with one hand.

The cover 3 preferably covers the entire valve cap 2 such that it fully surrounds the valve cap 2. Preferably, all openings 20 of the valve cap 2 are fully covered by the cover 3 in order to make sure that the valve unit is protected during transport from being contaminated. Furthermore, such a full cover provides a tamper-proof arrangement which ensures that the gas cylinder 1 is fully filled when it arrives at the respective customer and to make sure that nobody has tampered with the contents of the gas cylinder 1.

Preferably, the cover 3 includes at least one opening or cavity 34 at the position where the handle 24 of the valve cap 2 is located. In particular, the cover 3 preferably closely resembles the outer contours of the valve cap 2 particularly at the handle 24 such that the gas cylinder 1 can be easily carried by gripping it at the handle 24 even if the cover 3 is in place.

The cover 3, furthermore, includes a cut-out 36 at a position where the hook 22 of the valve cap 2 is provided such that the hook 22 can still be used.

As can be seen in Figure 1, the cover 3 closely resembles the outer contours of the valve cap 2. The close resemblance of the cover 3 to the valve cap 2 has, inter alia, the advantage that the cover 3 cannot be easily removed and that the cover 3 receives structural integrity from the directly supporting valve cap material.

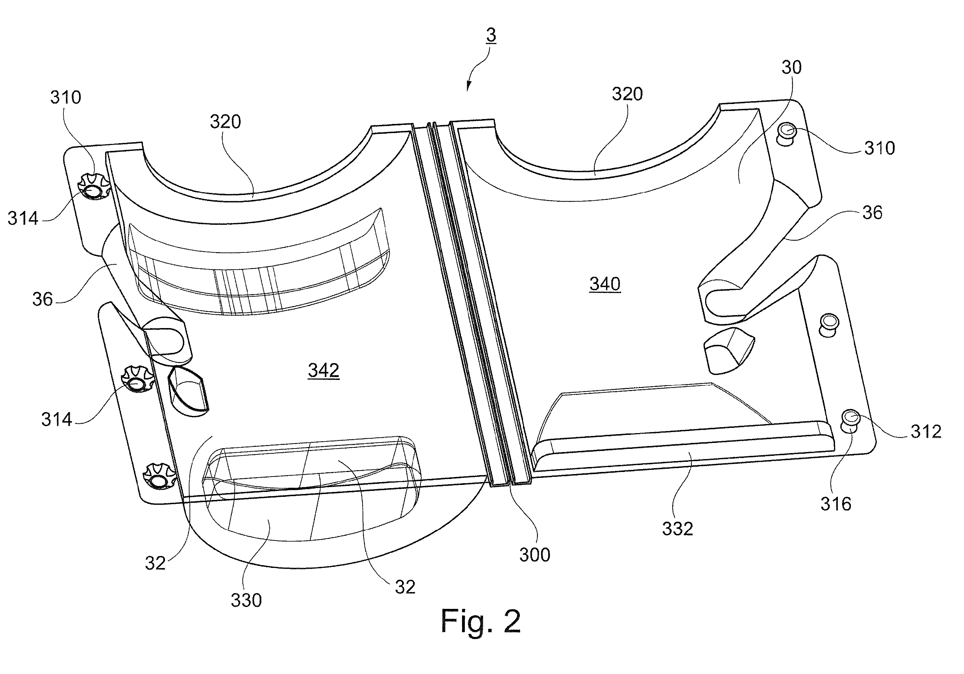

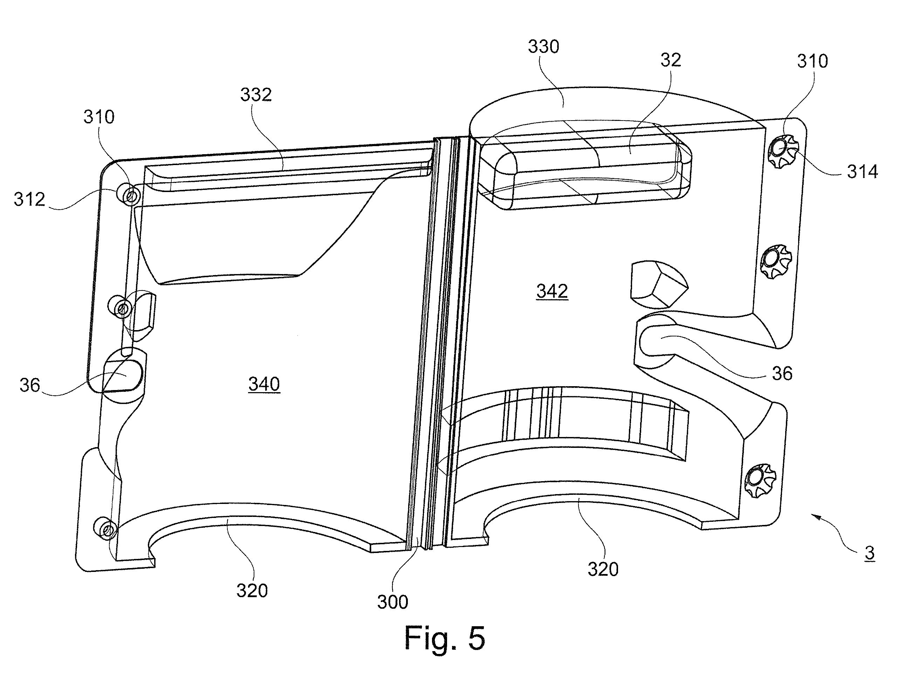

Figures 2 and 5 show the cover 3 in its opened state. The cover 3 comprises a first half-shell 30 and a second half-shell 32 which are connected with respect to one another by means of a foil hinge 300 and/or at least one tongue.

Preferably, the first half-shell 30, the second half-shell 32 as well as the foil hinge are manufactured by deep-drawing a suitable material or by casting the respective material integrally. In other words, the two half-shells and its connection are preferably made in one piece from a sheet of a suitable plastics material such as a sheet of PET (Polyethylene Terephthalate) or PVC (Polyvinyl Chloride). Other suitable materials include PCTFE (Polychlorotrifluoro ethylene) laminated to PVC, or COC (Cyclic Olefin Copolymers), or COP (Cyclic Olefin Polymers) which are provided in order to improve the moisture barrier, preferably in multilayered combinations with PP (Polypropylene), PE (Polyethylene), or PETg (glycol-modified polyethylene terephthalate).

In an alternative, an injection molding process could be used for manufacturing the cover 3 as well.

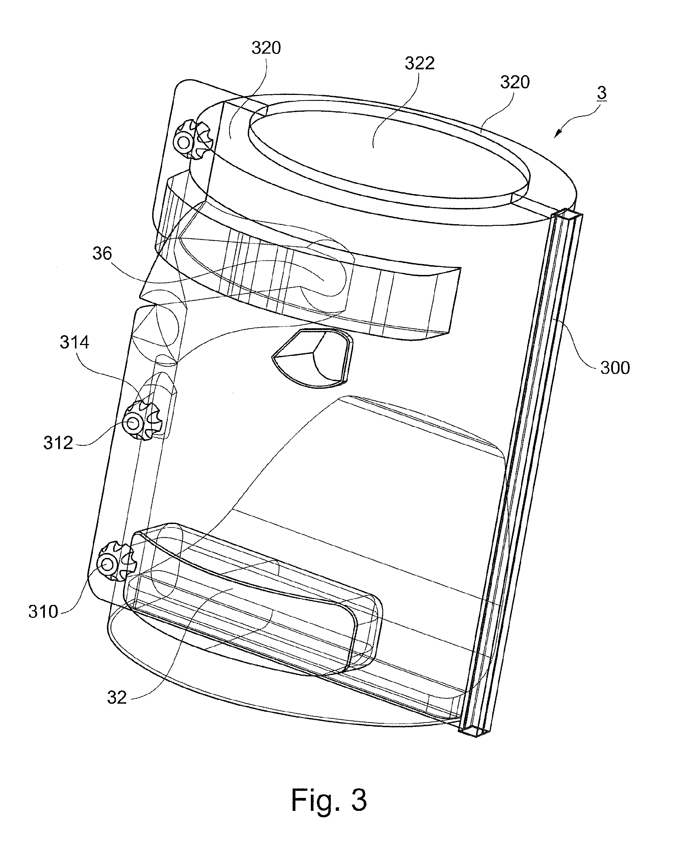

The foil hinge 300 is used to pivot the two half-shells 30, 32 with respect to one another in order to close the cover 3 in the form as it is shown in Figure 3 and to form the cavity which is dimensioned to receive the valve cap 2 of the gas cylinder 1. In an alternative or further development which is not shown in the Figures the two half-shells 30, 32 are connected via tongues.

In an alternative which is not shown in the Figures, the cover 3 comprises more than two parts which form the cavity when connected. Preferably, the at least two parts are interconnectible by means of at least one tamper-proof connector to form a tamper-proof cover and/or wherein at least two parts are connected via a foil hinge and/or a tongue.

In order to close the cover 3 shown in the Figures and keep it in the closed condition, fastening means 310 are provided. The fastening means 310 comprise a bolt 312 as well as a corresponding receiving opening 314. The bolt 312 is dimensioned to be received in the receiving opening 314 such that a fixed connection is formed upon insertion of the bolt 312 into the opening 314.

In an alternative or further development the connection can also be provided in the form of a clip, a thermal welding, an agrafe, a cramp, a staple, a plastic collar and/or an adhesive band.

In the preferred embodiment shown in the Figures, the connection cannot be reopened without destroying the material of the cover 3. In this respect, the dome-shaped material surrounding the openings 314 interacts with a bead 316 of the bolts 312 such that when the bolts 312 are inserted through the openings 314, the dome-shaped material is elastically extended and retracted after the bead 316 has passed the material. Accordingly, the dome-shaped material surrounding the opening 314 rests behind the bead 316. When it is attempted to reopen the cover 3, the dome-shaped portions surrounding the opening 314 are destroyed. In other words, the connection is formed as a frangible connection such that it results in a tamper-proof arrangement.

The tamper-proof arrangement is particularly suitable for gas cylinders which are used to distribute medical gases or industrial gases because it enables the end-user to determine whether the gas cylinder has been tampered with such that the end user can make sure that the gas cylinder contains the expected gas.

In order to be received at a handle portion of the valve cap of a gas cylinder, a cavity 32 is present in the cover 3. When considering Figure 2, it becomes immediately apparent that this cavity 32 also serves to carry the gas cylinder singlehanded.

The interconnection of the bolts 312 with the dome-shaped material surrounding the openings 314 leads to a tamper-proof sealing of the cover once it has been set in place enclosed on the valve cap 2. In fact, as can be seen in Figure 3, the cover 3 can only be removed by destroying either the connection between the bolts 312 and the dome-shaped material around the openings 314, by destroying the foil hinge 300, or by destroying any other portion of the material of the cover 3. In other words, the cover 3 cannot be removed without being destroyed.

Accordingly, the cover 3 serves as a tamper-proof member in the sense that a user can take from the condition of the cover 3 whether it has been tampered with the gas cylinder or whether it is in its intended state.

The two half-shells 30, 32 include, in their bottom portion, a collar 320, wherein this collar 320 is intended to be inserted below the lower portion of the valve cap 2 of the gas cylinder 1 when the cover 3 is in its intended, closed state. The position of the collar 320 of the cover 3 in the closed state of the cover 3 is schematically shown in Figure 1.

The collar portions 320 combine to a full collar once the two half-shells 30, 32 are folded upon one another. This can be clearly seen in Figure 3, in which the bottom portion of the cover 3 including the collar 320 is situated in the upper portion of the Figure. In fact, the opening 322 which remains at the inner diameter of the collar 320 is intended to receive the valve unit but is intended to have a diameter smaller than the lower diameter of the valve cap 2 of the gas cylinder 1.

The top portion of the cover 3 is intended to close the cavity which is formed when the two half-shells 30, 32 are folded upon one another. To this end, an upper portion 330 of the cover 3 is provided in the second half-shell 32, which is intended to cooperate with a protrusion 332 in the first half-shell 30 in order to close the cover 3 when the two half-shells 30, 32 are folded upon one another. The protrusion 332 is intended to be slid below the upper portion 330 of the cover 3 once the first and second half-shells are folded upon one another.

The inner cavities 340 and 342 of the respective half-shells 30 and 32 are preferably shaped such that they closely resemble the outer contours of the valve cap 2 of the gas cylinder 1. By this measure, not only an efficient way of transporting the gas cylinder 1 can be achieved but also a higher degree of resistance of the cover can be achieved.

## Claims

1. Cover (3) for a valve cap (2) of a gas cylinder (1), wherein the cover (3) includes a cavity (340, 342) which is dimensioned to receive at least a portion of the valve cap (2) of the gas cylinder (1),
**characterized in that**
the cover (3) is made from a substantially rigid material.

2. Cover (3) according to claim 1, wherein the cover (3) is made from a thermoformable material, preferably a thermoformable plastics material, preferably made from PET (Polyethylene Terephthalate), PVC (Polyvinyl Chloride), PCTFE (Polychlorotrifluoro Ethylene) laminated to PVC, COC (Cyclic Olefin Copolymers), COP (Cyclic Olefin Polymers), preferably in multilayered combinations with PP (Polypropylene), PE (Polyethylene), or PETg (glycol-modified Polyethylene Terephthalate) or the cover (3) is injection molded.

3. Cover (3) according to claim 1 or 2, wherein the cover (3) is provided in the form of a blister pack and/or a clamshell pack.

4. Cover (3) according to any one of the preceding claims, wherein the cover includes a tamper-proof connecting mechanism (310, 312, 314, 316), preferably in form of a frangible connecting arrangement, more preferably in the form of a clip, a thermal welding, an agrafe, a cramp, a staple, a plastic collar and/or an adhesive band.

5. Cover (3) according to any one of the preceding claims, comprising two half-shells (30, 32) which form the cavity (340, 342) for receiving portions of the valve cap (2) when folded upon themselves.

6. Cover (3) according to claim 5, wherein the two half-shells (30, 32) are interconnectible by means of at least one tamper-proof connector (310, 312, 314, 316) to form a tamper-proof cover.

7. Cover (3) according to claim 5 or 6, wherein the two half-shells (30, 32) are connected via a foil hinge (300) and/or a tongue.

8. Cover (3) according to any one of claims 1 to 4, comprising more than two parts which form the cavity when connected.

9. Cover (3) according to claim 8, wherein at least two parts are interconnectible by means of at least one tamper-proof connector, preferably in the form of a clip, a thermal welding, an agrafe, a cramp, a staple, a plastic collar and/or an adhesive band, to form a tamper-proof cover and/or wherein at least two parts are connected via a foil hinge (300) and/or a tongue.

10. Cover (3) according to any one of the preceding claims, wherein the cavity is formed such that it fully surrounds the valve cap (2) of the gas cylinder (1) when applied to the valve cap (2).

11. Cover (3) according to any one of the preceding claims, wherein the cover (3) is made in one piece, preferably by thermoforming a thermoformable plastics sheet or by injection molding.

12. Cover (3) according to any one of the preceding claims, wherein a handle portion (32) of the cavity, resembling a handle portion (24) of the valve cap (2), is formed in the cover (3).

13. Cover (3) according to any one of the preceding claims, wherein a collar (320) is formed in the cover (3) which is intended to be arranged at a bottom portion of the valve cap (2).

14. Cover (3) according to claim 13, wherein the collar (320) defines an opening (322) having a diameter smaller than the largest diameter of the valve cap (2).

15. Cover (3) according to any one of the preceding claims, wherein the cover is formed to hermetically seal off the valve cap (2) of the gas cylinder (1) when being applied to the valve cap (2).

16. Cover (3) according to any one of the preceding claims, wherein openings for receiving a hook or any other fastening means situated at the valve cap of the gas cylinder are provided.
